## Europäisches Patentamt

(19) **European Patent Office**

**Office européen des brevets**

(11) Veröffentlichungsnummer: **0 216 315**

**B1**

(12) # EUROPÄISCHE PATENTSCHRIFT

(45) Veröffentlichungstag der Patentschrift:
15.11.89

(51) Int. Cl.⁴: **C 07 C 45/50, C 07 C 47/02**

(21) Anmeldenummer: 86112907.0

(22) Anmeldetag: 18.09.86

(54) Verfahren zur Herstellung von Aldehyden.

(30) Priorität: 26.09.85 DE 3534314

(43) Veröffentlichungstag der Anmeldung:
01.04.87 Patentblatt 87/14

(45) Bekanntmachung des Hinweises auf die Patenterteilung:
15.11.89 Patentblatt 89/46

(84) Benannte Vertragsstaaten:
AT BE DE FR GB IT

(56) Entgegenhaltungen:
EP-A-0 157 316
EP-A-0 158 246
EP-A-0 163 234
DE-A-3 135 127
DE-A-3 235 030
DE-A-3 415 968
DE-B-2 627 354
FR-A-2 473 504

Die Akte enthält technische Angaben, die nach
dem Eingang der Anmeldung eingereicht wurden
und die nicht in dieser Patentschrift enthalten sind.

(73) Patentinhaber: HOECHST AKTIENGESELLSCHAFT,
Postfach 80 03 20, D-6230 Frankfurt am Main 80
(DE)

(72) Erfinder: Bahrmann, Helmut, Dr. Dipl.- Chem.,
Rohstrasse 48, D-4236 Hamminkeln 3 (DE)
Erfinder: Konkol, Werner, Dr. Dipl.- Chem.,
Lützowstrasse 40 a, D-4200 Oberhausen 11 (DE)
Erfinder: Weber, Jürgen, Dr. Dipl.- Chem.,
Bunsenstrasse 17, D-4200 Oberhausen 11 (DE)
Erfinder: Bach, Hanswilhelm, Dr. Dipl.- Chem.,
Alleestrasse 56, D-4100 Duisburg 11 (DE)
Erfinder: Bexten, Ludger, Dr. Dipl.- Chem., Im
Freihof 9, D-4224 Hünxe (DE)

EP 0 216 315 B1

LIBER, STOCKHOLM 1989

**Beschreibung**

Die vorliegende Erfindung betrifft ein Verfahren zur Herstellung von Aldehyden durch Umsetzung von olefinischen Verbindungen mit Wasserstoff und Kohlenmonoxid bei erhöhter Temperatur und erhöhtem Druck in homogener Phase und in Gegenwart eines Rhodium sowie aromatische Phosphine im molaren Überschuß enthaltenden Katalysatorsystems und Abtrennung des Katalysators vom Reaktionsprodukt.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzter Zeit Rhodium zunehmende Bedeutung. Im Gegensatz zu Kobalt gestattet Rhodium die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre Phosphine oder Phospite. Ihre Anwendung ermöglicht, den Reaktionsdruck auf Werte unter 30 MPa zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefine mit bis zu etwa 5 Kohlenstoffatomen im Molekül, beschritten werden.

Bei der Hydroformylierung Langkettiger Olefine oder olefinischer Verbindungen mit funktionellen Gruppen werden Produkte mit hohem Siedepunkt gebildet, die sich destillativ vom homogen gelösten Rhodium-Komplexkatalysator nicht abtrennen lassen. Die thermische Belastung des Destillationsgutes führt durch Dickölbildung zu erheblichen Verlusten an Wertprodukten und an Katalysator durch Zersetzung der Rhodiumkomplexverbindungen.

Die Abtrennung des Katalysators auf thermischen Wege wird durch Anwendung in Wasser löslicher Katalysatorsysteme vermieden. Derartige Katalysatoren sind z. B. in der DE-B2-2 627 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei der Verfahrensvariante einfach durch Trennung von wäßriger und organischer Phase, d.h. ohne Destillation und damit ohne zusätliche thermische Verfahrensschritte. Ein weiteres Merkmal dieser Arbeitsweise ist, daß mit hoher Selektivität aus endständigen Olefinen n-Aldehyde und nur in ganz untergeordnetem Maße iso-Aldehyde gebildet werden. Neben sulfonierten Triarylphosphinen werden als Komplexbestandteile wasserlöslicher Rhodiumkomplexverbindungen auch carboxylierte Triarylphosphine eingesetzt.

Die DE-A1-3 235 030 betrifft ein Verfahren zur Herstellung sulfonierter Triarylphosphine durch Umsetzung von Triarylphosphinen mit Oleum, Verdünnen des Sulfonierungsgemisches mit Wasser und Extraktion der sulfonierten Triarylphosphine mittels wasserunlöslicher Amine, die in einem organischen, wasserunlöslichen Solvens gelöst sind. Infolge einer chemischen Umsetzung bilden sich Aminsalzgemische sulfonierter Triarylphosphine, die von dem verdünnten Sulfonierungsgemisch abgetrennt werden. Durch Behandlung mit der wäßrigen Lösung einer Base werden die Aminsalze gespalten und die als Katalysatorbestandteil verwendbaren, sulfonierten Triarylphosphine gelangen als wasserlösliche Salze in die Wasserphase.

Eine Verwendung der Aminsalzgemische als Katalysatorbestandteil zur Hydroformylierung von Olefinen ist in der DE-A1-3 235 030 nicht erwähnt.

Der Einsatz wasserlöslicher Katalysatoren hat sich ausgezeichnet bei der Hydroformylierung niederer Olefine, insbesondere Ethylen und Propylen, bewährt. Setzt man höhere Olefine wie Hexen, Octen oder Decen ein, gehen der Umsatz und/oder die Selektivität der Reaktion zu n-Verbindungen merklich zurück. Die Wirtschaftlichkeit der Umsetzung in technischem Maßstab ist dann häufig nicht mehr gegeben.

Es bestand daher die Aufgabe, ein Verfahren zu entwickeln, das es erlaubt, olefinische Verbindungen, deren Hydroformylierungsprodukte einen relativ hohen Siedepunkt aufweisen oder nicht destillierbar sind, zu hydroformylieren und den Katalysator nach Hydroformylierung unter schonenden Bedingungen möglichst vollständig abzutrennen.

Diese Aufgabe wird gelöst durch ein Verfahren zur Herstellung von Aldehyden durch Umsetzung von olefinischen Verbindungen mit Wasserstoff und Kohlenmonoxid bei 100 bis 170°C und 0,3 bis 45 MPa in homogener Phase und in Gegenwart eines Rhodium sowie aromatische Phosphine im molaren Überschuß enthaltenden Katalysatorsystems und Abtrennung des Katalysators, dadurch gekennzeichnet, daß man als aromatische Phosphine in organischen Medien lösliche, in Wasser unlösliche Salze sulfonierter oder carboxylierter Triarylphosphine einsetzt, das Hydroformylierungsprodukt mit einer verdünnten wäßrigen Lösung einer Base behandelt und die wäßrige, das Katalysatorsystem enthaltende Phase abtrennt.

Das erfindungsgemäße Verfahren vereinigt die Vorteile bekannter Hydroformylierungsprozesse, ohne deren Nachteile zu besitzen. Einerseits gestattet es die Hydroformylierung olefinischer Verbindungen in homogener Phase und stellt damit einen hohen Umsatz sicher. Andererseits ermöglicht es eine schonende Spaltung und weitgehende Abtrennung des Katalysators vor einer weiteren Verarbeitung des Reaktionsproduktes durch Reindestillation, Hydrierung oder Oxidation.

Geeignete olefinische Verbindungen sind Verbindungen mit 6 bis 20 Kohlenstoffatomen, die eine oder mehrere olefinische Doppelbindungen besitzen. Sie können aliphatisch, cycloaliphatisch oder araliphatisch sein. Beispiele für aliphatische Verbindungen sind geradkettige und/oder verzweigte Olefine mit endständiger und/oder innenständiger Lage der Doppelbindung. Besonders geeignet sind geradkettige Olefine mit 6 bis 12 Kohlenstoffatomen wie n-Hexen-1, n-Hepten-1, n-Octen-1, n-Nonen-1, n-Decen-1, n-Undecen-1 und n-Dodecen-1, acyclische Terpene und verzweigte Olefine wie Diisobutylen, Tripropylen, Tetrapropylen und Dimersol.

Beispiele für aliphatische Diene sind 1,3-Butadien, 1,5-Hexadien und 1,9 Decadien. Beispiele für cycloaliphatische Einsatzstoffe sind Cyclohexen, Cycloocten, Cyclooctadien, Dicyclopentadien und cyclische Terpene wie Limonen, Pinen, Camphoren und Bisabolen. Beispielhaft für araliphatische Olefine steht Styrol.

Als olefinische Verbindungen mit funktionellen Gruppen sind Acrylsäurederivate insbesondere die Ester, Methacrylsäurederivate insbesondere die Ester, Allylverbindungen insbesondere die Alkohole und Ester Vinylverbindungen insbesondere die Ester und Ether, Cyanoverbindungen, insbesondere Acrylnitril sowie Acroleinderivate zu nennen.

Sekundäre Amine $NHR_2$ sollen insgesamt 10 bis 40, insbesondere 12 bis 34, bevorzugt 14 bis 26 Kohlenstoffatome aufweisen. Besonders wirksam sind Di-2-ethylhexylamin, Diisooctylamin, Diisononylamin und Dicyclohexylamin. Tertiäre Amine $NR_3$ sollen insgesamt 15 bis 50, insbesondere 18 bis 42, bevorzugt 21 bis 39 Kohlenstoffatome aufweisen. Besonders tauglich sind Triisooctylamin, Tri-n-octylamin, Triisononylamin, Triisodecylamin und Triisotridecylamin.

Das Katalysatorsystem besteht aus Salzen sulfonierter oder carboxylierter Triarylphosphine, die zwar in organischen Medien löslich, aber in Wasser unlöslich sind, und dem über das Phosphoratom komplex gebundenen Rhodium. Die Kationen der Salze weisen die Gruppierung $(NR_2H_2)+$ und/oder $(NR_3H)+$ auf, wobei R für Alkylreste mit 4 bis 12 Kohlenstoffatomen, für Aryl- oder Cycloalkylreste mit 6 bis 12 Kohlenstoffatomen steht.

Besonders geeignet sind die Kationen folgender Amine: Dicylohexylamin (Siedepunkt Sdp 256°C), Di-2-ethylhexylamin (Sdp 281°C), Triisooctylamin (Sdp. 340°C), Tri-n-octylamin (Sdp. 360°C), Triisononylamin (Sdp. 345°C) und Triisodecylamin (Sdp. 360°C).

Die Hydroformylierung der olefinischen Verbindungen erfolgt bei 100 bis 170 °C und 0,3 bis 45 MPa (3 bis 450 bar) in Gegenwart von 5 bis 500 ppm Rhodium, vorzugsweise 10 bis 150 ppm Rhodium bezogen auf olefinische Verbindung. Die Salze der sulfonierten oder carboxylierten Triarylphosphine werden im Verhältnis 5 : 1 bis 200 : 1, vorzugsweise 10 : 1 bis 100 : 1 (Mol Triarylphosphinsalz je g-Atom Rhodium) eingesetzt.

Die olefinische Verbindung kann als solche oder in Lösung der Hydroformylierung zugeführt werden. Geeignete Lösungsmittel sind Cyclohexan, Methylcyclohexan, Toluol und Xylol.

Die Reaktionsbedingungen hängen auch von der Art der olefinischen Verbindung ab. So lassen sich reaktionsfähige Einsatzstoffe bereits bei relativ niedrigen Temperaturen und Drücken in Gegenwart recht geringer Mengen an Katalysator umsetzen, während reaktionsträgere Stoffe entsprechend schärfere Reaktionsbedingungen erfordern. Beispiele für reaktive Olefine sind 1-Hexen, 1-Octen, 1-Decen, 1-Dodecen, Cyclohexen, Styrol. Beispiele für reaktionsträge Olefine sind 4-n-Octen, Tripropylen, Tetrapropylen, Dicyclopentadien, Limonen.

Das eingesetzte Synthesegas weist die molare Zusammensetzung $CO : H_2 = 1 : 1$ auf. Es ist jedoch auch möglich, von diesem Verhältnis abzuweichen. Im allgemeinen gelangt ein $CO : H_2$ Gemisch von 5 : 1 bis 1 : 5 zum Einsatz. Als besonders brauchbare sulfonierte Triarylphosphine haben sich mono, di- oder trisulfoniertes Triphenylphosphin, nämlich $(C_6H_5)_2PC_6H_4SO_3H$, $C_6H_5P(C_6H_5SO_3H)_2$ bzw. $P(C_6H_4SO_3H)_3$ erwiesen.

Aber auch Mischungen mono-, di- und trisulfonierter Triphenylphosphine sind als Katalysatorkomponente geeignet.

Nach der Hydroformylierung wird das Reaktionsgemisch mit einer verdünnten wäßrigen Lösung einer wasserlöslichen Base behandelt.

Die zur Spaltung der in organischen Medien löslichen, in Wasser unlöslichen Salze sulfonierter oder carboxylierter Triarylphosphine benötigten wasserlöslichen Basen müssen hinreichend alkalisch sein, um den gewünschten pH-Wert bei der Extraktion des Hydroformylierungsgemisches mit der verdünnten, wäßrigen Lösung der Base einzustellen. Diese Anforderung erfüllen die Alkali- und Erdalkalihydroxide, es können aber auch wäßrige Tetraalkylammoniumhydroxid-Lösungen verwendet werden. Die Konzentration der wasserlöslichen Base beträgt 0,05 bis 10 Gew.-%, insbesondere 1 bis 3 Gew.-%, bezogen auf wäßrige Lösung.

Durch Vermischen beider Phasen bei Temperaturen $\leq 70°C$, vorzugsweise $\leq 40°C$, werden aus den $(NR_2H_2)+$-, $(NR_3H)+$-Salzen, die entsprechenden sekundären oder tertiären Amine freigesetzt, gleichzeitig wird ein wasserlösliches Salz des sulfonierten oder carboxylierten Triarylphosphins gebildet. Dieses gelangt durch Extraktion in die wäßrige Phase und wird zusammen mit dem komplex am Phosphor gebundenen Rhodium abgetennt.

Während dieser Extraktion soll der pH-Wert des Gemisches beider Phasen $\geq 8$, vorzugsweise $\geq 8,5$ betragen. Im allgemeinen empfiehlt sich, einen pH-Bereich von 8 bis 13,7 vorzugsweise 8,5 bis 10,5 einzuhalten.

Die Extraktion ist einfach auszuführen. Infolge der verringerten thermischen Belastung wird einerseits die Desaktivierung und thermische Zersetzung und damit die Schädigung des Katalysatorsystems herabgesetzt, andererseits wird die Bildung unerwünschter Nebenprodukte, die aus dem Hydroformylierungsprodukt entstehen, vermindert.

Die Rückgewinnungsquote ist bereits im ersten Extraktionsschritt hoch und liegt über 70 Gew.-% bezogen auf eingesetztes Rhodium. Durch mehrfache Extraktion kann dieses Resultat weiter verbessert werden.

Organische und wäßrige Phase trennen sich schnell und vollständig voneinander.

Um die Entmischung der Phasen zu beschleunigen, zentrifugiert man gegebenenfalls und separiert anschließend die obere organische Phase von der unteren wäßrigem Phase. Besonders bewährt haben sich hierfür Separatoren mit Koaleszierelementen.

Die Anwesenheit von Luft oder Sauerstoff soll soweit als möglich vermieden werden, um eine Oxidation des gelösten Phosphins zu verhindern. Wird das dreiwertige Phosphoratom oxidiert, so verschlechtert sich die Rückgewinnung des Rhodiums bei der Extraktion. In diesem Falle behilft man sich damit, dem zu extrahierenden Material frisches Triarylphosphin zuzusetzen und anschließend zu extrahieren. Nach Abtrennung der Rhodium und Triarylphosphin enthaltenden Wasserphase wird das Hydroformylierungsprodukt - falls erforderlich - mehrfach mit kaltem Wasser gewaschen, um noch vorhandene basische Anteile zu beseitigen. Diese Reinigung muß sorgfältig erfolgen, um basisch katalysierte Nebenreaktionen, z. B. Aldolisierungen, während der Hydrierung zu vermeiden.

Ein weiterer Vorteil des erfindungsgemäßen Verfahrens besteht darin, daß aus der wäßrigen Rhodium enthaltenden Phase ohne großen Aufwand der aktive Hydroformylierungskatalysator wieder zurückgewonnen werden kann. Man säuert den Extrakt z. B. mit Mineralsäuren bis zu einem pH-Wert von $\approx$ 1 und extrahiert die wäßrige Phase mit einem in einem organischen Lösungsmittel (z. B. Benzol oder Toluol) gelösten sekundären bzw. tertiären Amin. Dabei bildet sich das ursprünglich eingesetzte Aminsalz des sulfonierten oder carboxylierten Triarylphosphins zurück. Da dieses in Wasser nicht löslich ist, geht es zusammen mit dem komplex gebundenen Rhodium in die organische Phase über. Der so gewonnene Reextrakt kann direkt als Hydroformylierungskatalysator wieder eingesetzt werden. Falls erforderlich ergänzt man noch Rhodium und/oder den Phosphor (III) Liganden.

Das Verfahren kann auch kontinuierliche durchgeführt werden.

In den folgenden Beispielen wird die Erfindung näher beschrieben.

## Versuch 1

Herstellung des Triisooctylammonium-Salzes von TPPTS

Die Sulfonierung von Triphenylphosphin mittels Oleum und die sich hieran anschließende Weiterverarbeitung ist in der DE-OS-3 235 030 beschrieben.

In Anlehnung an diese Arbeitsweise wird Triphenylphosphin bei Raumtemperatur mit Oleum umgesetzt und das entstandene Gemisch durch Zusatz von kaltem Wasser hydrolisiert. Anschließend fügt man eine Lösung von Triisooctylamin in Toluol zu und rührt etwa 30 Minuten. Nach Beendigung des Rührens wird die untere Schwefelsäure enthaltende Wasserphase abgetrennt. Durch Zusatz von 3 %-iger wäßriger Natronlauge wird ein pH-Wert von 4,6 eingestellt, die Wasserphase abgetrennt und verworfen. Anschließend wird die toluolische Lösung noch zweimal mit Wasser gewaschen. Zur Vermeidung unerwünschter Oxidation des Phosphins wird unter striktem Sauerstoffausschluß gearbeitet.

Auf diese Weise werden zwei Phosphin-Mischungen I und II hergestellt, deren analytische Daten in der Tabelle 1 zusammengestellt sind.

Die nachfolgend verwendeten Abkürzungen bedeuten:

TPPDS   : Triphenylphosphindisulfonsäure-salz
TPPTS   : Triphenylphosphintrisulfonsäure-salz
TPPODS: Triphenylphosphinoxiddisulfonsäure-salz
TPPOTS: Triphenylphosphinoxidtrisulfonsäure-salz
TPPSTS: Triphenylphosphinsulfidtrisulfonsäure-salz
HPLC     : High pressure liquid chromatography

## Tabelle 1

(Werte mittels HPLC-Analyse als Natriumsalze bestimmt)

|  | Phosphin-[1] Mischung I | Phosphin-[1] Mischung II |
|---|---|---|
| TPPDS | 1,714 Gew.-% | 0,677 Gew.-% |
| TPPTS | 8,087 " | 2,80 " |
| TPPODS | 0,205 " | 0,108 " |
| TPPOTS | 0,809 " | 0,894 " |
| P(III)[2] | 0,165 mol/kg | 0,075 mol/kg |

4

1) Aminsalzlösung in Toluol
2) jodometrisch bestimmt

## Beispiel 1

Im folgenden wird die Hydroformylierung olefinischer Verbindungen beschrieben.

Ein ausreichend bemessener Autoklav, der eine Rührvorrichtung, Thermometer und Einlaßstutzen zur Zufuhr von Synthesegas besitzt, wird mit Synthesegas oder Stickstoff sauerstoffrei gespült. Anschließend wird die olefinische Verbindung, das Salz des sulfonierten Triarylphosphins (wie unter Versuch 1 beschrieben) hergestellt, Rhodium (in Form von Rhodium-2-Ethylhexanoat) und gegebenenfalls ein Lösungsmittel eingefüllt. Der Inhalt des Autoklaven wird durch Aufpressen von Synthesegas ($CO : H_2 = 1 : 1$) auf Druck gebracht und unter Rühren aufgeheizt. Der Verlauf der Reaktion wird durch Probenahmen während der Umsetzung überwacht. Die Reaktionsbedingungen sind der nachfolgenden Tabelle 2 zu entnehmen.

## Tabelle 2

| Olefin: | Cyclohexen | Dimersol | Diisobutylen | Dicycloclopen-tadien | n-Hexen-1 | Limonen | n-Decen-1 |
|---|---|---|---|---|---|---|---|
| Olefinmenge (g) | 300 | 300 | 300 | 1000 | 2400 | 600 | 300 |
| Toluol (g) | 300 | 300 | 300 | - | - | 300 | 300 |
| Rhodium (besogen auf Olefin | 10 ppm | 10 ppm | 30 ppm | 60 ppm | 10 ppm | 150 ppm | 50 ppm |
| TPPTS/TIOA* Lösung (g) | 18,7 | 18,7 | 17,8 | 187 | 150 | 561 | 93,5 |
| mmol P(III) | 3,1 | 3,1 | 2,9 | 30,9 | 24,8 | 92 | 15,4 |
| Rh:P(III) (molar) | 1 : 100 | 1 : 100 | 1 : 33 | 1 : 53,3 | 1 : 108 | 1 : 106 | 1 : 100 |
| Druck (MPa) | 27 | 27 | 27 | 27 | 2,5 | 27 | 27 |
| Temperatur °C | 130 | 130 | 130 | 130 | 130 | 130 | 130 |
| Reaktions-zeit (h) | 6 | 6 | 6 | 6 | 2,5 | 6 | 6 |
| Umsatz (%) | 100 | 81 | 99 | 100 | 77 | 98 | 99 |
| Ausbeute (%) | -** | 75 | 79 | -** | -** | 64 | 92 |

* Triphenylphosphin-trisulfonat-Triisooctylamin gelöst in Toluol
** nicht betstimmt

## Beispiel 2

Abtrennung des Rhodium enthaltenden Katalysatorsystems

Das aus der Hydroformylierung entstammende Reaktionsgemisch wird unter Rühren und Luftausschluß solange mit verdünnter wäßriger Lauge (3 Gew.-%-ige NaOH) versetzt, bis der gewünschte pH-Wert erreicht wird. Man stellt das Rühren ein und wartet die Phasentrennung ab. Gegebenenfalls zentrifugiert man das Gemenge aus organischem Produkt und wäßriger Lösung, um die Abtrennung der Wasserphase zu beschleunigen.

In der Wasserphase befindet sich der größte Teil des Rhodium enthaltenden Katalysators in Form des Natriumsalzes des trisulfonierten Triarylphoshins. Um die Abtrennung von im organischen Produkt noch vorhandenem Katalysator zu vervollständigen, kann die oben beschriebene Extraktion ein oder mehrfach wiederholt werden, wobei der einzustellende pH-Wert von Mal zu Mal ansteigt. Abschließend wird die organische Phase gründlich mit Wasser und gegebenenfalls mit verdünnter Säure gewaschen, um noch vorhandene basische Reste zu entfernen.

Die Bedingungen der Extraktion und ihre Ergebnisse sind in der nachfolgenden Tabelle 3 zusammengestellt.

**Tabelle 3** Teil 1

**Rhodium-Abtrennung aus dem rohen Hydroformylierungsprodukt**

| Hydrofor- myliertes Olefin | Cyclohexen | Dimersol | Diiso- butylen | Dicyclopen- tadien | n-Hexen-1 | Limonen | n-Decen-1 |
|---|---|---|---|---|---|---|---|
| Menge | 426 g | 452 g | 458 g | 1164 g | 1038 g | 100 g | 533 g |
| Temperatur | 10°C | 6°C | 6°C | 6°C | 10°C | 10°C | 10°C |
| **1. Abtrennung** | | | | | | | |
| pH-Wert | 11,0 | 11,3 | 9,1 | 12,6 | 10,0 | 10,0 | 10,0 |
| Zusatz von 3 %-iger wäßriger NaOH | 67,8 g | 47,6 g | 39,8 g | 165 g | 220 g | 340 g | 55 g |
| Rhodium (% der Theorie) in wäßriger Phase | 29,0 | 28,0 (76,4)[1] | 37,0 (61,7)[1] | 55,7[1] | 52,3 | 78,7[1] | 65,9[1] |
| **Teil 2** **2. Abtrennung** | | | | | | | |
| pH-Wert | - | - | - | - | 13,1 | 13,7 | 13,0 |
| Zusatz von 3 %-iger wäßriger NaHO | - | - | - | - | 47,3 | 23,5 | 18 |
| Wasserwäsche (Menge Was- serzusatzt) | - | - | - | - | 300 g | 299 g | 190 g |
| Neutralisation [2] | - | - | - | - | 15 g[2] | 25,5[2] | 3 g[2] |
| Rhodium (% oder Theorie) in wäßrigen Phasen gesamt | 29 | 28 (76,4[1] | 37,0 (61,7)[1] | 69,5 (79,9)[1] | 67,2 (65)[1] | 82,3[1] | 92(92)[1] |

[1] bezogen auf im Hydroformylierungsprodukt ermitteltes Rhodium
()[1] bezogen auf im Hydroformylierungsprodukt ermitteltes Rhodium
[2] verdünnte $H_2SO_4$
(0,5 %-ig - 1,5 %-ig Wasser)

**Beispiel 3**

Rückgewinnung des Rhodium enthaltenden Katalysators und Überführung in seine aktive Form

Der wäßrige Extrakt wird mit verdünnter Säure (z. B. $H_2SO_4$ 1 %-ig) angesäuert bis etwa pH 1 bis 2 erreicht wird. Die Reextraktion des aktiven Katalysators erfolgt mit einer Lösung des betreffenden ursprünglich verwendeten Amins in einem organischen Lösungsmittel. Es wird intensiv durchmischt und dabei das Amin mit der sauren Wasserphase umgesetzt. Das gebildete Aminsalz ist wasserunlöslich und geht in das organische Lösungsmittel über. Somit liegt der Hydroformylierungskatalysator in der ursprünglich verwendeten Form vor. Aufgetretene Verluste an Rhodium und/oder Phosphinligand werden durch Zusatz von frischem Rhodium und/oder Ligand ausgeglichen.

Setzt man diesen aufgearbeiteten Katalysator erneut in die Hydroformylierung ein, so werden nahezu die gleichen Ergebnisse wie in den Tabellen 2 und 3 aufgeführt erzielt.

**Patentansprüche**

1. Verfahren zur Herstellung von Aldehyden durch Umsetzung von olefinischen Verbindungen mit Wasserstoff und Kohlenmonoxid bei 100 bis 170°C und 0,3 bis 45 MPa in homogener Phase und in Gegenwart eines Rhodium sowie aromatische Phosphine im molaren Überschuß enthaltenden Katalysatorsystems und

EP 0 216 315 B1

Abtrennung des Katalysators, dadurch gekennzeichnet, daß man als aromatische Phosphine in organischen Medien lösliche, in Wasser unlösliche Salze sulfonierter oder carboxylierter Triarylphosphine einsetzt, das Hydroformylierungsprodukt mit einer verdünnten wäßrigen Lösung einer Base behandelt und die wäßrigen das Katalysatorsystem enthaltende Phase, abtrennt.

2. Verfahren nach Anspruch 1, dadurch gekennzeichnet, daß die in organischen Medien löslichen, in Wasser unlöslichen Salze sulfonierter oder carboxylierter Triarylphosphine als Kation $(NR_2H_2)^+$ und/oder $(NR_3H)^+$ enthalten, wobei R für Alkylreste mit 4 bis 12 Kohlenstoffatomen, für Aryl- oder Cycloalkylreste mit 6 bis 12 Kohlenstoffatomen steht.

3. Verfahren nach Anspruch 1 und 2, dadurch gekennzeichnet, daß die Hydroformylierung der olefinischen Verbindung in Gegenwart von 5 bis 500 ppm Rhodium bezogen auf eingesetzte olefinische Verbindung erfolgt und die Salze der sulfonierten oder carboxylierten Triarylphosphine in einem molaren Verhältnis von 5 : 1 bis 200 : 1 bezogen auf Rhodium eingesetzt werden.

4. Verfahren nach Anspruch 1 bis 3, dadurch gekennzeichnet, daß die Hydroformylierung der olefinischen Verbindung in Gegenwart von 10 bis 150 ppm Rhodium bezogen auf eingesetzte olefinische Verbindung erfolgt und die Salze der sulfonierten oder carboxylierten Triarylphosphine in einem molaren Verhältnis von 10 : 1 bis 100 : 1 bezogen auf Rhodium eingesetzt werden.

5. Verfahren nach Anspruch 1 bis 4, dadurch gekennzeichnet, daß das sulfonierte Triarylphosphin mono-, di- oder trisulfoniertes Triphenylphosphin ist.

6. Verfahren nach Anspruch 1 bis 5, dadurch gekennzeichnet, daß das sulfonierte Triarylphosphin aus Mischungen mono-, di- und trisulfonierter Triphenylphosphine besteht.

7. Verfahren nach Anspruch 1 bis 6, dadurch gekennzeichnet, daß die Abtrennung des Katalysatorsystems mittels der wasserlöslichen Base bei einer Temperatur von $\leq 70°C$ und einem pH-Wert von $\geq 8$ durchgeführt wird.

8. Verfahren nach Anspruch 1 bis 7, dadurch gekennzeichnet, daß die Abtrennung des Katalysatorsystems mittels der wäßrigem Lösung der wasserlöslichen Base bei einer Temperatur $\leq 40°C$ und einem pH-Wert von $\geq 8,5$ durchgeführt wird.

9. Verfahren nach Anspruch 1 bis 8, dadurch gekennzeichnet, daß als wasserlösliche Base Alkali-, Erdalkalihydroxide oder wäßrigen Tetraalkylammoniumhydroxid-Lösung verwendet werden.

10. Verfahren nach Anspruch 1 bis 9, dadurch gekennzeichnet, daß die Abtrennung des Hydroformylierungsproduktes und der wäßrigen Lösung durch Zentrifugieren oder in einem Separator mit Koaleszierelementen herbeigeführt wird.

11. Verfahren nach Anspruch 1 bis 10, dadurch gekennzeichnet, daß man die das Katalysatorsystem enthaltende, vom Hydroformylierungsprodukt abgetrennte wäßrige Phase ansäuert, mit einem in einem wasserunlöslichen organischen Lösungsmittel gelösten Amin der Formel $NHR_2$ bzw. $NR_3$, wobei R für Alkylreste mit 4 bis 12 Kohlenstoffatomen, für Aryl- oder Cycloalkylreste mit 6 bis 12 Kohlenstoffatomen steht, behandelt und nach Abtrennung der wäßrigen Phase die organische Phase wieder in die Hydroformylierung einsetzt.

**Claims**

1. A process for the preparation of aldehydes by the reaction of olefinic compounds with hydrogen and carbon monoxide at 100 to 170°C and 0.3 to 45 MPa in homogeneous phase and in the presence of a catalyst system containing rhodium and aromatic phosphines in molar excess and separation of the catalyst, characterised in that salts of sulfonated or carboxylated triarylphosphines which are soluble in organic media but insoluble in water are employed as aromatic phosphines, the hydroformylation product is treated with a diluted aqueous solution of a base and the aqueous phase containing the catalyst system is separated.

2. A process according to claim 1, characterised in that the salts of sulfonated or carboxylated triarylphosphines which are soluble in organic media but insoluble in water contain $(NR_2H_2)^+$ and/or $(NR_3H)^+$ as cations, whereby R denotes alkyl groups with 4 to 12 carbon atoms, respectively aryl or cycloalkyl groups with 6 to 12 carbon atoms.

3. A process according to claims 1 and 2, characterised in that the hydroformylation of the olefinic compound takes place in the presence of 5 to 500 ppm rhodium, related to the olefinic compound employed, and the salts of the sulfonated or carboxylated triarylphosphines are employed in a molar ratio of 5 : 1 to 200 : 1, related to the rhodium.

4. A process according to claims 1 and 3, characterised in that the hydroformylation of the olefinic compound takes place in the presence of 10 to 150 ppm rhodium, related to the olefinic compound employed, and the salts of the sulfonated or carboxylated triarylphosphines are employed in a molar ratio of 10 : 1 to 100 : 1, related to the rhodium.

5. A process according to claims 1 to 4, characterised in that the sulfonated triarylphosphine is mono, di or trisulfonated triphenylphosphine.

6. A process according to claims 1 to 5, characterised in that the sulfonated triarylphosphine consists of a mixture of mono, di and trisulfonated triphenylphosphines.

7. A process according to claims 1 to 6, characterised in that the catalyst system is separated by means of

7

the water-soluble base at a temperature of $\leqslant 70°$C and a pH value of $\geqslant 8$.

8. A process according to claims 1 to 7, characterised in that the catalyst system is separated by means of the aqueous solution of the water-soluble base at a temperature of $\leqslant 40°$C and a pH value of $\geqslant 8.5$.

9. A process according to claims 1 to 8, characterised in that an alkali, alkaline earth hydroxide or aqueous tetraalkylammonium hydroxide solution is employed as a water-soluble base.

10. A process according to claims 1 to 9, characterised in that the hydroformylation product and aqueous solution are separated by means of centrifugal force or in a separator with coalescing agents.

11. A process according to claims 1 to 10, characterised in that the aqueous phase containing the catalyst system and separated from the hydroformylation product is acidified, treated with an amine dissolved in a water-insoluble organic solvent, said amine having the formula $NHR_2$ or $NR_3$, where R denotes alkyl groups with 4 to 12 carbon atoms, aryl or cycloalkyl groups with 6 to 12 carbon atoms and after separation of the aqueous phase the organic phase is re-employed in the hydroformylation.

**Revendications**

1. Procédé de préparation d'aldéhydes par réaction de composés oléfiniques avec l'hydrogène et l'oxyde de carbone à des températures de 100 à 170°C et des pressions de 0,3 à 45 MPa en phase homogène et en présence d'un système catalyseur contenant du rhodium et des phosphines aromatiques en excès molaire, et séparation du catalyseur, caractérisé en ce que l'on utilise en tant que phosphines aromatiques des sels solubles dans les milieux organiques mais insolubles dans l'eau de triarylphosphines sulfonées ou carboxylées, on traite le produit d'hydroformylation par une solution aqueuse diluée d'une base et on sépare la phase aqueuse contenant le système catalyseur.

2. Procédé selon la revendication 1, caractérisé en ce que les sels solubles dans les milieux organiques mais insolubles dans l'eau des triarylphosphines sulfonées ou carboxylées contiennent en tant que cations $(NR_2H_2)^+$ et/ou $(NR_3H)^+$, R représentant des groupes alkyle en C4-C12, aryle ou cycloalkyle en C6 - C12.

3. Procédé selon les revendications 1 et 2, caractérisé en ce que l'hydroformylation du composé oléfinique est effectuée en présence de 5 à 500 ppm de rhodium par rapport au composé oléfinique mis en oeuvre et en ce que l'on utilise les sels des triarylphosphines sulfonées ou carboxylées à un rapport molaire de 5 : 1 à 200 : 1 avec le rhodium.

4. Procédé selon les revendications 1 à 3, caractérisé en ce que l'hydroformylation du composé oléfinique est effectuée en présence de 10 à 150 ppm de rhodium par rapport au composé oléfinique mis en oeuvre et en ce que l'on utilise les sels des triarylphosphines sulfonées ou carboxylées à un rapport molaire de 10 : 1 à 100 : 1 avec le rhodium.

5. Procédé selon les revendications 1 à 4, caractérisé en ce que la triarylphosphine sulfonée consiste en triphénylphosphine mono-, di- ou tri-sulfonée.

6. Procédé selon les revendications 1 à 5, caractérisé en ce que la triarylphosphine sulfonée consiste en mélanges de triphénylphosphines mono-, di- et tri-sulfonées.

7. Procédé selon les revendications 1 à 6, caractérisé en ce que la séparation du système catalyseur à l'aide de la base soluble dans l'eau est effectuée à une température inférieure ou égale à 70°C et à un pH supérieur ou égal à 8.

8. Procédé selon les revendications 1 à 7, caractérisé en ce que la séparation du système catalyseur à l'aide de la solution aqueuse de la base soluble dans l'eau est réalisée à une température inférieure ou égale à 40°C et à un pH supérieur ou égal à 8,5.

9. Procédé selon les revendications 1 à 8, caractérisé en ce que l'on utilise en tant que bases solubles dans l'eau des hydroxydes alcalins ou alcalino-terreux ou des solutions aqueuses d'hydroxydes de tétraalkylammonium.

10. Procédé selon les revendications 1 à 9, caractérisé en ce que la séparation du produit d'hydroformylation et de la solution aqueuse est réalisée par centrifugation ou dans un séparateur à éléments de coalescence.

11. Procédé selon les revendications 1 à 10, caractérisé en ce que l'on acidifie la phase aqueuse séparée du produit d'hydroformylation, et contenant le système catalyseur, on la traite par une amine de formule $NHR_2$ ou $NR_3$, R représentant des groupes alkyle en C4 - C12, aryle ou cycloalkyle en C6 - C12 en solution dans un solvant organique insoluble dans l'eau et, après séparation de la phase aqueuse, on réutilise la phase organique à l'hydroformylation.